# EUROPEAN PATENT SPECIFICATION

(11) **EP 2 086 600 B1**
(45) Date of publication and mention of the grant of the patent: **26.10.2016**
(21) Application number: 07870838.5
(22) Date of filing: 01.11.2007
(51) Int. Cl.: A61L 31/10, A61L 31/14, A61L 31/16, A61L 27/34, A61L 27/54, A61L 29/08, A61L 29/16

(54) **STENTS WITH DRUG ELUTING COATINGS**
STENTS MIT ARZNEIMITTEL FREISETZENDEN ÜBERZÜGEN
ENDOPROTHÈSES VASCULAIRES RECOUVERTES D'UN ENDUIT PERMETTANT D'ÉLUER DES AGENTS PHARMACOLOGIQUES

(30) Priority: 03.11.2006 US 856873 P
(43) Date of publication of application: 12.08.2009
(73) Proprietor: Boston Scientific Limited, Hamilton HM11 (BM)
(72) Inventor: MALONE, Anthony, Oranhill Oranmore (IE); O'CONNOR, Tim, Claregalway Co. Galway (IE); MCMORROW, Dave, Fort Lorenzo Galway (IE); FLANAGAN, Aiden, Kilcolgan Galway (IE)
(74) Representative: Peterreins Schley
(86) International application number: PCT/US2007/023221
(87) International publication number: WO 2008/066656

(56) References cited:
- JP-A- 58 199 187
- US-A1- 2003 069 631
- US-A1- 2005 037 047
- US-A1- 2006 035 854
- US-B1- 6 656 506
- H. Henning Winter: "Gel Point" In: "Encyclopedia of Polymer Science and Technology", 15 October 2003 (2003-10-15), John Wiley & Sons, Inc., Hoboken, NJ, USA, XP055007628, ISBN: 978-0-47-144026-0 DOI: 10.1002/0471440264.pst476, * the whole document *

## Description

### 2. FIELD OF THE INVENTION

The present invention generally relates to coated medical devices (e.g., stents) comprising one or more surfaces having disposed thereon a plurality of microparticles comprising an active pharmaceutical ingredient (API) and a polymer. Methods for preparing the coated medical devices and methods of using the coated medical devices to treat or prevent stenosis or restenosis in a subject, preferably a human, are also provided.

### 3. BACKGROUND OF THE INVENTION

Cardiovascular disease is a leading cause of death in the developed world. Patients having such disease usually have narrowing or closing (stenosis) in one or more arteries. The use of stents in the treatment of cardiovascular disease is well known. Stents are typically delivered in a contracted state to the treatment area within a lumen, where they are then expanded. Balloon-expandable stents expand from a contracted state by deforming in response to a force exerted upon the stent body by a balloon that is inflated within the stent's lumen. Once expanded within a body lumen, the stent body is strong enough to resist any contracting force exerted by the body lumen wall so that the stent maintains its expanded diameter. In contrast, self-expanding stents have resilient bodies that exert a radial expansion force when the stent is compressed. A self-expanding stent that is deployed within a body lumen will expand until the body lumen wall exerts a compressive force against the stent that is equal to the radial expansion force.

The use of balloon-expandable and self-expanding stents, however, may have the disadvantage of causing additional trauma to a body lumen upon deployment of the stent. Typically, a stent is expanded within a body lumen so that the diameter of the stent is greater than that of the body lumen. As a result, the edges of the ends of stent may be pressed into the wall of body lumen, stressing the wall to the point of creating additional trauma, i.e., cutting or tearing of the body lumen wall. This trauma may ultimately lead to restenosis (re-narrowing) in the areas of the body lumen adjacent the ends of the stent.

Recently, various types of drug-coated stents have been used for the localized delivery of active pharmaceutical ingredients (APIs) to the wall of a body lumen to further prevent restenosis. The APIs used as part of the stent coating typically have one or more therapeutic activities such as antithrombotic activity, antiproliferative activity, anti-inflammatory activity, vasodilatory activity, or lipid-lowering activity. Generally, APIs are adhered to the stent surface in admixture with a carrier polymer. The polymer provides several functions which are important in assuring the stent's performance once it is inserted in the patient. First, whereas many APIs are hydrophobic and would otherwise fail to bind to bare metal stents, the polymer effectively adheres the APIs to the stent. Second, the polymer controls the release of the APIs from the stent to provide a sustained, localized delivery of the APIs from the stent. Certain APIs, e.g., cytostatic agents, if provided on the stent surface in an uncoated form, would result in a local concentration of APIs that would exceed the APIs' therapeutically active range and could be toxic. Polymer carriers, in effect, can reduce the local concentration of the API and provide a therapeutically useful concentration of the agent. Moreover, in applications where a more soluble API is coated on the stent, the polymer can control the API's release rate by minimizing the rapid dissolution of the API into the bloodstream, and thus, prevent the undesired elimination of the API from the desired treatment area.

While the polymer provides the drug-coated stent with several important functions, the use of the polymer also burdens the stent with certain disadvantages. Often, coating the API with a polymer can result in drug entrapment within the polymer coating so that the API diffuses from the stent to the area to be treated too slowly and/or at too low a concentration to be therapeutically useful. Moreover, conventional coating methods typically use a continuous phase coating such as a liquid carrier polymer phase to dispose the API on the stent. Such methods often result in disposing an excess amount of polymer on the stent surface. The presence of excess polymer is generally considered to be detrimental to tissue recovery, and a bare metal stent is believed to promote better vascular healing than a stent having a polymer finish.

In applications where the stent manufacturer intends to disperse a low concentration of a potent API on the stent, and particularly where a low concentration of the API on only certain portions of the stent is to be dispersed, the stent typically contains a disproportionately high ratio of polymer to API. As noted supra, the excess polymer impedes the recovery of the tissue surrounding the stent.

Accordingly, there is a need for a medical device, e.g., a stent, which is prepared by coating methods that effectively adhere APIs to the device surface and provide controlled release of such agents from the device, yet minimize the amount of polymer disposed on the device surface.

### 4. SUMMARY OF THE INVENTION

To achieve the aforementioned objectives, the inventors has invented an insertable or implantable medical device as defined in appended claim 1.

In another aspect, the invention relates to a method for making the coated medical device of the invention as defined in the appended claims.

According to the invention, the polymer is a thermosetting polymer having a gel point. In such embodiments, the pre-treating step of the method for making the coated medical device of the invention can comprise pre-heating the medical device, or a surface of the medical device.

In certain embodiments of the method, the pre-treating step of the method for making the coated medical device of the invention comprises masking a portion of the surface with a masking agent. Masking allows certain portions of the stent to remain uncoated after the contacting step.

The invention also relates to a medical device, e.g., a stent, prepared by the above-described methods. The invention further is useful in a method of treating stenosis or restenosis, comprising inserting or implanting the coated medical device in a subject in need of such treatment.

### 4.1 DEFINITIONS

As used herein, the term "continuous phase coating" refers to a polymeric carrier phase which when disposed on an outer surface of a medical device exists as a continuous layer.

As used herein, the term "hydrophilic" refers to the characteristics of being readily absorbable or soluble in water, e.g., having polar groups (in which the distribution of electrons is uneven, enabling it to take part in electrostatic interactions) that readily interact with water, and/or having an affinity for water.

As used herein, the term "hydrophobic" refers to the characteristics of not being readily absorbable or soluble in water, e.g., being adversely affected by water, and/or having little or no affinity for water.

As used herein, the term "microparticles" refers to an isolated population of particles having an average particle diameter of less than 100 µm. The term also includes an isolated population of particles having an average particle diameter of less than 1 µm, i.e., nanoparticles.

As used herein the term, "microparticulate phase coating" refers to a polymeric carrier phase which is present on an outer surface of a medical device as discrete microparticles containing a polymer and an API.

As used herein, the prefix "nano-" means 10"9.

As used herein, the terms "subject" and "patient" are used interchangeably. As used herein, a subject is preferably a mammal such as a non-primate {e.g., cows, pigs, horses, cats, dogs, rats, etc.) or a primate (e.g., monkey and human), most preferably a human.

As used herein, the term "therapeutically effective amount" refers to that amount of API sufficient to inhibit cell proliferation, contraction, migration, hyperactivity, or address other conditions. A therapeutically effective amount may refer to the amount of API sufficient to delay or minimize the onset of symptoms associated with cell proliferation, contraction, migration, hyperactivity, or address other conditions. A therapeutically effective amount may also refer to the amount of API that provides a therapeutic benefit in the treatment or management of certain conditions such as stenosis or restenosis and/or symptoms associated with stenosis or restenosis.

### 5. FIGURES

**Figure 1A** shows one embodiment of a coated medical device of the invention 1 having a plurality of microparticles 2 disposed on a device surface Ia.
**Figure 1B** shows one embodiment of a microparticle 2 encapsulating particles of an active pharmaceutical ingredient (API) 2a within a matrix of polymer 2b.
**Figure 1C** shows another embodiment of a microparticle 2 encapsulating a particle of an API 2a within a matrix of polymer 2b.
**Figure 2** is a flow chart depicting one embodiment of a coating method of the invention.

### 6. DETAILED DESCRIPTION OF THE INVENTION

The present invention relates to a medical device (e.g., a stent) comprising one or more surfaces having disposed thereon a plurality of microparticles comprising an active pharmaceutical ingredient (API) and a polymer. Specifically, the microparticles are disposed on the surface in a microparticulate phase coating, i.e., as discrete microparticles in the absence of a continuous phase coating. The coated medical device of the invention effectively adheres a plurality of the microparticles to its surface, and allows controlled release of the APIs therein from the microparticles to a desired treatment area by using a minimal amount of polymer.

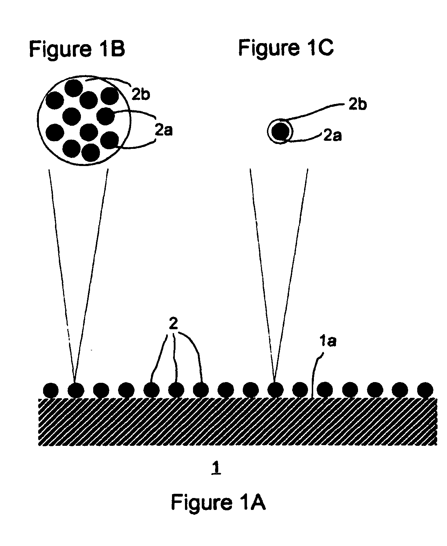

Figure IA, for example, shows one embodiment of a coated medical device of the inventio 1 having a device surface Ia. Disposed on the device surface Ia are a plurality of microparticles 2. Figure IB depicts one embodiment of a single microparticle 2 viewed in cross-section, where the microparticle 2 contains particles of API 2a encapsulated within a matrix of polymer 2b. Figure 1C depicts another embodiment of a single microparticle 2 viewed in cross-section, where the microparticle 2 contains a particle of API 2a encapsulated within a matrix of polymer 2b. The coated medical devices are discussed in more detail in Section 5.1 infra.

The invention also provides methods for making a coated medical device that include pre-treating a surface of the medical device to promote adhesion between the surface and the microparticles. The microparticles are disposed on the pre-treated surface with a fluidized bed of microparticles. As such, the APIs are coated on the stent in the absence of a continuous phase coating.

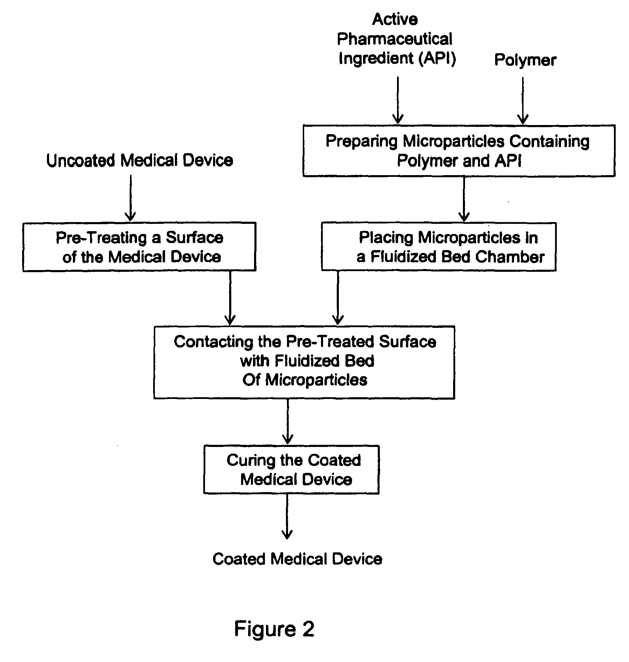

Figure 2, for example, is a flow chart depicting a specific embodiment of a coating method of the invention. In this embodiment, microparticles are prepared from an API and a polymer, and placed in a fluidized bed chamber. A surface of the uncoated medical device is pre-treated and then the pre-treated surface is contacted with the fluidized bed of
microparticles to coat the surface. The coated medical device is then cured to further adhere the microparticles to the device surface to form the finished, coated medical device. The coating methods of the invention are discussed in more detail in Section 5.2 infra.

While not being bound by any specific theory, the inventors believe that discrete microparticles provide optimal platforms from which to deliver APIs from coated medical devices since the requirements of effective cohesion of APIs to device surfaces, and controlled release of APIs from those surfaces can be achieved with minimal amount of polymer. Medical devices prepared according to the coating methods of the invention achieve efficient and consistent release of one or more APIs from the coated devices to inhibit cell proliferation, contraction, migration, hyperactivity and/or other conditions.

For clarity of disclosure, and not by way of limitation, the detailed description of the invention is divided into the subsections which follow.

### 6.1 COATED MEDICAL DEVICES

### 6.1.1 Coating Embodiments for Medical Devices

The coated medical device of the invention can be coated with microparticles that can contain the same or different types of APIs. In one embodiment, the device is coated with microparticles that contain the same type of API. The API can be disposed on the device using a single distribution of microparticles uniformly prepared with the same polymer. Alternatively, the API can be disposed on the device in more than one distribution of microparticles that may vary by API concentration or by the choice of the polymer used to form the microparticles.

In other embodiments, the coated medical device of the invention can be coated with microparticles that contain different types of APIs. For example, in specific embodiments, the medical device is coated with a first distribution of microparticles each containing a first API and a second distribution of microparticles each containing a second API. The polymer used to form the microparticles in the first and second distribution of microparticles can be the same or different. Where different polymers are used to form the different distributions of microparticles, appropriate selection of the polymers for each distribution allows specific control of the release profile for each type of API, so that drug delivery for each type of API can be individually optimized.

The entire surface of the coated medical device of the invention can be uniformly coated or, alternatively, different portions of the device surface can be differentially coated. In some embodiments, a medical device can have a first surface and a second surface. The first surface has disposed thereon a first distribution of microparticles each containing an API and a polymer, whereas the second surface is free of microparticles, or has disposed thereon a second distribution of microparticles that is different from the first distribution. In certain embodiments, the microparticles used to form the first and second distributions are identical, but the second distribution of microparticles is disposed on the second surface at a concentration, i.e., a second concentration, that is different from the first distribution of microparticles. In other embodiments having first and second distributions of microparticles, the second distribution of microparticles can contain a different concentrations of the same type of API, different types of APIs, different polymers of the same or different types of APIs, or any combination thereof.

By way of example, in preparing drug-coated stents, a first surface of the stent may comprise the abluminal side of the stent and the second surface of the stent may comprise the luminal side of the stent. The abluminal side of the stent, i.e., the first surface, can have disposed thereon a first distribution of microparticles each containing an API and a polymer. The luminal side of the stent, i.e., the second surface, may be free of microparticles, or have disposed thereon a second distribution of microparticles that is different from the first distribution. The second distribution may differ from the first distribution in terms of API or polymer concentration, type of API or polymer, or any combination thereof, as described supra.

In specific embodiments, the invention relates to a medical device having a first surface and a second surface, where the first surface has a first distribution of microparticles disposed thereon at a concentration of from 0.2 to 5 //g/mm2, and preferably from 0.5 to 1.5 µg/mm2 (e.g., about 1 //g/mm2) on the basis of the API. The methods of the invention effectively adhere the microparticles to the first surface at low API concentrations by using less polymer than would be needed by conventional coating methods which rely on a continuous phase coating (e.g., a continuous polymer phase coating) to adhere the microparticles to the first surface. Therefore, in one aspect, the invention provides an advantageous method of localizing a lower concentration of the API, e.g., from 0.2 to 5 µg/mm2 on the basis of the API, to specific portions of the device surface with minimized polymer utilization. Since the device contains a minimal amount of polymer, the device, e.g., a stent, when inserted or implanted in a subject, provides more effective tissue recovery than devices prepared with higher polymer loadings.

### 6.1.2 Microparticles on Medical Devices

The microparticles used in the coated medical device of the invention comprise an API and a polymer. In specific embodiments, the microparticles are prepared according to the methods described in Section 5.2.1 *infra.*

In some embodiments, one or more APIs are encapsulated into a microparticle. In a preferred embodiment, each microparticle comprises one API. APIs suitable for encapsulating in the microparticles are described in Section 5.1.2.1 *infra.*

Preferably, the API comprises at least 5 wt.% of the microparticles to ensure that the coated medical device of the invention contains a minimal amount of polymer to provide adequate API release rates from the device and to promote tissue recovery as discussed *supra.* For instance, in specific embodiments, the API comprises at least 10 wt.%, least 20 wt.%, or at least 30 wt.% of the microparticle. One skilled in the art would realize that to some extent the specific concentration of APIs encapsulated within the microparticles will vary based upon the nature of the API. For instance, for certain APIs, the therapeutic window, the desired rate of release, and the API's affinity for the polymer within the microparticle will dictate the specific concentrations of API to be encapsulated in the microparticle.

In certain embodiments, the polymer-containing microparticle is capable of providing sustained release of one or more APIs over a time period. The time period for release of an API from the microparticle ranges from 1 hour, 2 hours, 3 hours, 4 hours, 5 hours, 6 hours, 12 hours, 1 day, 2 days, 3 days, 4 days, 5 days, 6 days, 1 week, 2 weeks, 3 weeks, 1 month, 2 months, 3 months, 4 months, 5 months, 6 months, 1 year, 2 years, or longer. Preferably, the time period for release of the API from the microparticle ranges from 1 hour to 24 months.

In certain embodiments, the microparticles, in addition to containing an API and polymer, can be labelled with, e.g., radioisotopes, antibodies, or colored with, e.g., dye.

### 6.1.2.1 Active Pharmaceutical Ingredients

In certain embodiments, the API encapsulated in the microparticles is useful for inhibiting cell proliferation, contraction, migration, hyperactivity, or addressing other conditions. The term "API" encompasses drugs, genetic materials, and biological materials. Non-limiting examples of suitable APIs include heparin, heparin derivatives, urokinase, dextrophenylalanine proline arginine chloromethylketone (PPack), enoxaprin, angiopeptin, hirudin, acetylsalicylic acid, tacrolimus, everolimus, rapamycin (sirolimus), amlodipine, doxazosin, glucocorticoids, betamethasone, dexamethasone, prednisolone, corticosterone, budesonide, sulfasalazine, rosiglitazone, mycophenolic acid, mesalamine, paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti thrombin antibodies, anti platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, antioxidants, probucol, antibiotic agents, penicillin, cefoxitin, oxacillin, tobranycin, angiogenic substances, fibroblast growth factors, estrogen, estradiol (E2), estriol (E3), 17-beta estradiol, digoxin, beta blockers, captopril, enalopril, statins, steroids, vitamins, taxol, paclitaxel, 2'-succinyl-taxol, 2'-succinyl-taxol triethanolamine, 2'-glutaryl-taxol, 2'-glutaryl-taxol triethanolamine salt, 2'-0-ester with N-(dimethylaminoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In a preferred embodiment, the API is taxol (e.g., Taxol®), or its analogs or derivatives. In another preferred embodiment, the API is paclitaxel. In yet another preferred embodiment, the API is an antibiotic such as erythromycin, amphotericin, rapamycin, adriamycin, etc.

The term "genetic materials" means DNA or RNA, including, without limitation, of DNA/RNA encoding a useful protein stated below, intended to be inserted into a human body including viral vectors and non-viral vectors.

The term "biological materials" include cells, yeasts, bacteria, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor- 1 (HIF-I), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (e.g., BMP-2, BMP-3, BMP-4, BMP-5, BMP-6 (Vgr-1), BMP-7 (PO-I), BMP-8, BMP-9, BMP-IO, BMP-I1, BMP- 12, BMP- 14, BMP-15, BMP- 16, etc.), matrix metalloproteinase (MMP), tissue inhibitor of matrix metalloproteinase (TIMP), cytokines, interleukin (e.g., IL-I, IL-2, IL-3, IL-4, IL-5, IL-6, IL-7, IL-8, IL-9, IL-IO, IL-11, IL- 12, IL-15, etc.), lymphokines, interferon, integrin, collagen (all types), elastin, fibrillins, fibronectin, vitronectin, laminin, glycosaminoglycans, proteoglycans, transferrin, cytotactin, cell binding domains (e.g., RGD), and tenascin. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source
(xenogeneic), genetically engineered, if desired, to deliver proteins of interest at the transplant site. The delivery media can be formulated as needed to maintain cell function and viability. Cells include progenitor cells (e.g., endothelial progenitor cells), stem cells (e.g., mesenchymal, hematopoietic, neuronal), stromal cells, parenchymal cells,
undifferentiated cells, fibroblasts, macrophage, and satellite cells.

### Other non-genetic APIs include:

- anti-thrombogenic agents such as heparin, heparin derivatives, urokinase, and PPack (dextrophenylalanine proline arginine chloromethyl ketone);
- anti-proliferative agents such as enoxaprin, angiopeptin, or monoclonal antibodies capable of blocking smooth muscle cell proliferation, hirudin, acetylsalicylic acid, tacrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid and mesalamine;
- anti-neoplastic/anti-proliferative/anti-miotic agents such as paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells; • vascular cell growth promoters such as growth factors, vascular endothelial growth factors (VEGF, all types including VEGF -2), growth factor receptors, transcriptional activators, and translational promoters;
- vascular cell growth inhibitors such as antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents; vasodilating agents; and agents which interfere with endogenous vasoactive mechanisms;
- anti-oxidants, such as probucol;
- antibiotic agents, such as penicillin, cefoxitin, oxacillin, tobramycin;
- macrolides such as sirolimus (rapamycin), everolimus, tacrolimus, pimecrolimus, and zotarolimus;
- angiogenic substances, such as acidic and basic fibroblast growth factors, estrogen including estradiol (E2), estriol (E3) and 17-beta estradiol; and
- drugs for heart failure, such as digoxin, beta-blockers, angiotensin-converting enzyme (ACE) inhibitors including captopril and enalopril, statins and related compounds. Preferred biologically active materials include anti-proliferative drugs such as steroids, vitamins, and restenosis-inhibiting agents. Preferred restenosis-inhibiting agents include microtubule stabilizing agents such as Taxol®, paclitaxel (i.e., paclitaxel, paclitaxel analogues, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-0-ester with N- (dimethylaminoethyl) glutamine, and 2'-O-ester with N-(dimethylaminoethyl) glutamide hydrochloride salt.

Other preferred APIs include nitroglycerin, nitrous oxides, nitric oxides, antibiotics, aspirins, digitalis, estrogen derivatives such as estradiol and glycosides.

In certain embodiments, the APIs for use in the coated medical devices of the invention can be synthesized by methods well known to one skilled in the art. Alternatively, the APIs can be purchased from chemical and pharmaceutical companies.

### 6.1.2.2 Polymers

The polymers suitable for use in the preparation of the microparticles of the present invention should be materials that are biocompatible and avoid irritation to body tissue. Other polymers which can be used include ones that can be dissolved and cured or polymerized on the medical device or polymers having relatively low melting points that can be blended with biologically active materials.

### 6.1.3 Types of Medical Devices

Uncoated medical devices serve as coating substrates upon which the microparticles are disposed in the coated medical devices of the invention. Medical devices that are useful in the present invention can be made of any biocompatible material suitable for medical devices in general which include without limitation natural polymers, synthetic polymers, ceramics, and metallics. Metallic material (e.g., niobium, niobium-zirconium, and tantalum) is more preferable. Suitable metallic materials include metals and alloys based on titanium (such as nitinol, nickel titanium alloys, thermo-memory alloy materials), stainless steel, tantalum, nickel-chrome, or certain cobalt alloys including cobalt-chromium-nickel alloys such as Elgiloy® and Phynox®. Metallic materials also include clad composite filaments, such as those disclosed in WO 94/16646.

Metallic materials may be made into elongated members or wire-like elements and then woven to form a network of metal mesh. Polymer filaments may also be used together with the metallic elongated members or wire-like elements to form a network mesh. If the network is made of metal, the intersection may be welded, twisted, bent, glued, tied (with suture), heat sealed to one another; or connected in any manner known in the art.

The polymer(s) useful for forming the medical device should be ones that are biocompatible and avoid irritation to body tissue. They can be either biostable or bioabsorbable. Suitable polymeric materials include without limitation polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for medical devices include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) co-polymer, polylactic acid, poly(ε-caprolactone), poly(β-hydroxybutyrate), polydioxanone, poly(γ-ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or cross-linking groups, e.g., Arg-Gly-Asp (RGD), in which the polymers retain their structural integrity while allowing for attachment of molecules, such as proteins, nucleic acids, and the like. The polymers may be dried to increase their mechanical strength. The polymers may then be used as the base material to form a whole or part of the medical device. Furthermore, although the invention can be practiced by using a single type of polymer to form the medical device, various combinations of polymers can also be employed. The appropriate mixture of polymers can be coordinated to produce desired effects when incorporated into a medical device.

Examples of the medical devices suitable for the present invention include, but are not limited to, stents, surgical staples, catheters (e.g., central venous catheters and arterial catheters), guidewires, cannulas, cardiac pacemaker leads or lead tips, cardiac defibrillator leads or lead tips, implantable vascular access ports, blood storage bags, blood tubing, vascular or other grafts, intra-aortic balloon pumps, heart valves, cardiovascular sutures, total artificial hearts and ventricular assist pumps, and extra-corporeal devices such as blood oxygenators, blood filters, hemodialysis units, hemoperfusion units and plasmapheresis units. In a preferred embodiment, the medical device is a stent.

Medical devices suitable for the present invention include those that have a tubular or cylindrical-like portion. The tubular portion of the medical device need not to be completely cylindrical. For instance, the cross-section of the tubular portion can be any shape, such as rectangle, a triangle, etc., not just a circle. Such devices include, without limitation, stents and grafts. A bifurcated stent is also included among the medical devices which can be fabricated by the method of the present invention.

Medical devices which are particularly suitable for the present invention include any kind of stent for medical purposes which is known to the skilled artisan. Suitable stents include, for example, vascular stents such as self-expanding stents and balloon expandable stents. Examples of self-expanding stents useful in the present invention are illustrated in United States Patent Nos. 4,655,771 and 4,954,126 issued to Wallsten and 5,061,275 issued to Wallsten et al. Examples of appropriate balloon-expandable stents are shown in United States Patent No. 5,449,373 issued to Pinchasik et al.

### 6.2 METHODS FOR MAKING THE MEDICAL DEVICES

### 6.2.1 Methods for Preparing the Microparticles

APIs can be encapsulated into polymeric microparticles by methods well known to one skilled in the art. Certain methods for encapsulating APIs into microparticles are described below to more particularly describe certain embodiments of the invention. The skilled artisan will recognize, however that other known methods for the encapsulation into microparticles can also be used.

In one embodiment, the API-encapsulated microparticles are prepared by phase inversion technology (PIN technology). Using this technology, a polymer is dissolved in an effective amount of a solvent. The API to be encapsulated is also dissolved or dispersed in the effective amount of the solvent. The polymer, the API and the solvent together form a mixture having a continuous phase. Then, the mixture is introduced into an effective amount of a nonsolvent to cause the spontaneous formation of the microencapsulated product, wherein the solvent and the nonsolvent are miscible. PIN technology has been described by, for example, Mathiowitz et al. in United States Patent No. 6,131,211 and United States Patent No. 6,235,224. In another embodiment, the API-encapsulated microparticles are prepared by rapid expansion of supercritical solutions (RESS) of API and polymer. In this method, the API and the polymer are both dissolved in a supercritical fluid {e.g., supercritical CO2) with or without a cosolvent, such as methanol or acetone. The solution is then released from a nozzle (de-pressurized), generating microparticles with a polymer coating on the surface. In RESS methods, the rapid depressurization of the supercritical solution causes a substantial lowering of the solvent power of CO2 leading to very high supersaturation of solute, precipitation, nucleation and particle growth. This method works best where the API and polymer are very soluble in the supercritical fluid. For instance, J. W. Tom et al. discloses a RESS method to make biocompatible and bioerodible polymer microspheres, mainly polyhydroxy acids including, poly(L-lactic acid) (L-PLA), poly(D,L-lactic acid),

(DL-PLA) and poly(glycolic acid) (PGA) which can be used for controlled delivery of APIs.

Nucleation of poly(L-lactic acid) from CO2 and CO2 -acetone mixtures produced microparticles and microspheres. See e.g., J. W. Tom et al, "Formation of bioerodible polymeric microspheres and microparticles by rapid expansion of supercritical solutions," Biotechnol. Prog. 1991;7(5):403-11.

In an alternative embodiment, the API-encapsulated microparticles are prepared by a gas anti-solvent (GAS) precipitation process. For GAS precipitation, the API and one or more polymers are dissolved in a conventional pharmaceutical solvent, which is immiscible with the supercritical fluid used, e.g., CO2. The resulting solution is then expanded using the supercritical fluid to precipitate the particles. Precipitation of the particles can be achieved by introducing the supercritical fluid into a batch of the API- and polymer-containing solution in a chamber, or by spraying the API- and polymer-containing solution into a chamber filled with the supercritical fluid. See, e.g., pp. 300-303 of S. D. Yeo et al,
"Formation of Polymer Particles with Supercritical Fluids: A Review," J. of Supercritical Fluids 34 (2005) 287-308.

In another embodiment, the API-encapsulated microparticles are prepared as particles from a gas saturated solution (PGSS). The PGSS method can be used to produce polymer composite materials containing the API as guest particles. The supercritical fluid is dissolved in molten polymer in the presence of insoluble particles of the API. Upon rapid decompression {e.g., depressurization through a nozzle) particles are formed by
precipitation, with the API distributed uniformly throughout the polymer matrix. Control of the particle size can be achieved through alterations in the pressure to which the polymer and API are exposed prior to depressurization. The PGSS method is generally described by, for example, Weidner et al in United States Patent No 6,056,791.

In still another embodiment, the invention relates to a method of encapsulating the API in the form of microparticles by spray freezing into liquids (SFL). In this embodiment, a mixture of the API, polymer and a liquid diluent is atomized into a cryogenic liquid to form frozen particles, which are then dried to provide the microparticles. Nanoparticles and microparticles of poorly water-soluble drugs, for example, have been produced using the SFL method. See, e.g., United States Application Publication No. 2003/0041602 A1 to Williams et al..

More particularly, the SFL method includes spraying the mixture of the API, polymer and liquid diluent (and optionally a surface modifier) through an insulating nozzle located at or below the level of a cryogenic liquid, wherein the spray generates frozen particles. The liquid diluent used to form the mixture with the API and polymer can be chosen from an aqueous, organic, or aqueous-organic co-solvent. When combined with the API and polymer, the diluent may form a mixture that is a solution, suspension or emulsion. The liquid diluent in the SFL method can be an aqueous solvent, such as water, one or more organic solvents, or a combination thereof. Suitable cryogenic fluids for the SFL method include materials (organic or inorganic) that remain liquid below the freezing point of water, and are non-reactive (do not undergo a chemical reaction with any of the components of the solution that is to be spray frozen). Non-limiting examples include:
carbon dioxide, nitrogen, ethane, isopentane, propane, helium, halocarbons, liquid ammonia and argon. The cryogenic liquid can be held statically in a vessel, or can be circulated through an appropriate vessel that is equipped with a filter to collect the particles that are formed.

The drying step of the SFL method includes lyophilizing the frozen particles or subliming the frozen particles at atmospheric pressure. See, e.g., Rogers et al, Pharm. Res. 20: 485-93 (2003)..

### 6.2.2 Methods of Coating the Medical Device

Coated medical devices can generally be coated by the following steps:
(a) placing microparticles comprising an API and a polymer in a fluidized bed chamber;
(b) pre-treating a surface of the medical device to promote adhesion between the surface and the microparticles; and
(c) contacting the pre-treated surface with the fluidized bed to dispose the microparticles on the surface.

The microparticles placed in the fluidized chamber may be prepared as described in Section 5.2.1 supra.

By pre-treating the surface of the medical device, the microparticles can be effectively adhered to a surface of the medical device. According to the invention where the microparticles are formed with a thermosetting polymer, the medical device or a portion of the surface of the medical device is heated. Upon contact with a heated surface of the medical device, the polymer component of the microparticles melts or partially melts, and the microparticles binds to the device surface. Heating of the device surface may occur prior to and/or simultaneous with contact with the microparticles. For instance, in specific embodiments of the invention, the medical device is heated while in contact with the fluidized bed of microparticles by radio frequency.

Moreover, a masking technique can be used to localize the adhesion of the microparticles to specific portions of the device surface. A portion of the device surface can be masked with a masking agent so that the masked portion remains uncoated after contact with the fluidized bed of
microparticles. By way of example, in coating stents, microparticles can be localized to specific struts or to only abluminal stent surfaces by masking the remainder of the stent surfaces. Masking agents include polymers which can be selectively removed through washing in a suitable solvent.

Contacting between the surface of the medical device and the microparticles is conducted by positioning the medical device to be coated in a fluidized bed of the microparticles. Typically, the microparticles are circulated in a dense dry particle distribution using a gas, such as an inert gas, e.g., nitrogen or argon. The coating methods of the invention are typically conducted under controlled conditions so that by adjusting the exposure time between the medical device and the microparticles, and/or by manipulating other fluid bed processing parameters, operators can achieve the desired coating density, thickness, and distribution on the device surface. In specific embodiments, the contacting is conducted in the absence of a continuous liquid phase.

The coating methods of the invention optionally include curing the coated medical device after contacting of the device surface with the fluidized bed of microparticles. In some embodiments the curing includes exposure of the device to a heating or cooling cycle to adhere the microparticles to the device surface. Alternatively, exposing the coated device surface to a spray or fine mist of a solvent that selectively and/or partially dissolves the polymer of the microparticles will also further fuse the microparticles to the stent surface. The coated medical device can also be subjected to a spinning or vacuum cycle to remove any excess or unattached microparticles during the curing step.

### 6.3 THERAPEUTIC USES

The invention is useful in the therapeutic use of the coated medical devices of the invention to address conditions such as stenosis or restenosis by inhibiting cell proliferation, contraction, migration or hyperactivity in a subject. The coated medical devices of the invention can be inserted or implanted into a subject in need thereof. In certain embodiments, the API used in the coated medical devices of the invention may be used to inhibit the proliferation, contraction, migration and/or hyperactivity of cells of the brain, neck, eye, mouth, throat, esophagus, chest, bone, ligament, cartilage, tendons, lung, colon, rectum, stomach, prostate, breast, ovaries, fallopian tubes, uterus, cervix, testicles or other reproductive organs, hair follicles, skin, diaphragm, thyroid, blood, muscles, bone, bone marrow, heart, lymph nodes, blood vessels, arteries, capillaries, large intestine, small intestine, kidney, liver, pancreas, brain, spinal cord, and the central nervous system. Preferably, the API is useful for inhibiting the proliferation, contraction, migration and/or hyperactivity of muscle cells, e.g., smooth muscle cells.

In another aspect, the API may be used to inhibit the proliferation, contraction, migration and/or hyperactivity of cells in body tissues, e.g., epithelial tissue, connective tissue, muscle tissue, and nerve tissue. Epithelial tissue covers or lines all body surfaces inside or outside the body. Examples of epithelial tissue include, but are not limited to, the skin, epithelium, dermis, and the mucosa and serosa that line the body cavity and internal organs, such as the heart, lung, liver, kidney, intestines, bladder, uterine, etc.

Connective tissue is the most abundant and widely distributed of all tissues. Examples of connective tissue include, but are not limited to, vascular tissue (e.g., arteries, veins, capillaries), blood (e.g., red blood cells, platelets, white blood cells), lymph, fat, fibers, cartilage, ligaments, tendon, bone, teeth, omentum, peritoneum, mesentery, meniscus, conjunctiva, dura mater, umbilical cord, etc. Muscle tissue accounts for nearly one-third of the total body weight and consists of three distinct subtypes: striated (skeletal) muscle, smooth (visceral) muscle, and cardiac muscle.

Examples of muscle tissue include, but are not limited to, myocardium (heart muscle), skeletal, intestinal wall, etc. The fourth primary type of tissue is nerve tissue. Nerve tissue is found in the brain, spinal cord, and
accompanying nerve. Nerve tissue is composed of specialized cells called neurons (nerve cells) and neuroglial or glial cells.

Preferably, the microparticles comprise one or more APIs useful for inhibiting muscle cell proliferation, contraction, migration or hyperactivity.

The coated medical devices of the invention may also be used to treat diseases that may benefit from decreased cell proliferation, contraction, migration and/or hyperactivity.

In particular, the APIs, such as paclitaxel, may be used to treat or prevent diseases or conditions that may benefit from decreased or slowed cell proliferation, contraction, migration or hyperactivity. In specific embodiments, the present invention inhibits at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, at least 10%, at least 5%, or at least 1% of cell proliferation, contraction, migration and/or hyperactivity.

The present invention further is useful in methods for treating or preventing stenosis or restenosis. In particular, the invention relates to methods for treating or preventing stenosis or restenosis by inserting or implanting a coated medical device of the invention into a subject. In such applications, the coated medical devices contain a therapeutically effective amount of the API.

As used herein, the terms "subject" and "patient" are used interchangeably. The subject can be an animal, preferably a mammal including a non-primate (e.g., a cow, pig, horse, cat, dog, rat, and mouse) and a primate (e.g., a monkey, such as a cynomologous monkey, chimpanzee, and a human), and more preferably a human. The subject can be a subject who had undergone a regimen of treatment (e.g., percutaneous transluminal coronary angioplasty (PTCA), also known as balloon angioplasty, and coronary artery bypass graft (CABG) operation).

The therapeutically effective amount of an API for the subject will vary with the subject treated and the API itself. The therapeutically effective amount will also vary with the condition to be treated and the severity of the condition to be treated. The dose, and perhaps the dose frequency, can also vary according to the age, gender, body weight, and response of the individual subject.

The present invention is useful alone or in combination with other treatment modalities. In certain cases, the subject can be receiving concurrently other therapies to treat or prevent stenosis or restenosis. In certain cases, the treatment further includes the administration of one or more immunotherapeutic agents, such as antibodies and immunomodulators, which include, but are not limited to, HERCEPTIN®, RITUXAN®, OVAREX™, PANG-REX®, BEC2, IMC-C225, VITAXIN™, CAMPATH® I/H, Smart MI95, LYMPHOCIDE™, Smart I DIO, ONCOL YM™, rituximab, gemtuzumab, or trastuzumab. In certain other embodiments, the treatment method further comprises hormonal treatment. Hormonal therapeutic treatments comprise hormonal agonists, hormonal antagonists (e.g., flutamide, tamoxifen, leuprolide acetate (LUPRON™), LH-RH antagonists), inhibitors of hormone biosynthesis and processing, steroids (e.g., dexamethasone, retinoids, betamethasone, Cortisol, cortisone, prednisone, dehydrotestosterone, glucocorticoids, mineralocorticoids, estrogen, testosterone, progestins), antigestagens (e.g., mifepristone, onapristone), and antiandrogens (e.g., cyproterone acetate).

In certain cases, the coated medical device of the invention is capable of providing sustained release of the APIs over a time period. The time period for release of a API from the device ranges from 1 hour, 2 hours, 3 hours, 4 hours, 5 hours, 6 hours, 12 hours, 1 day, 2 days, 3 days, 4 days, 5 days, 6 days, 1 week, 2 weeks, 3 weeks, 1 month, 2 months, 3 months, 4 months, 5 months, 6 months, 1 year, 2 years, or longer. Preferably, the time period for release of the API from the device ranges from 1 hour to 24 months.

In specific cases, as discussed in Section 5.1.2.2 supra, the coated medical device of the invention contains microparticles having polymers that are capable of changing their conformation upon a change in the microenvironment of the polymer. In such embodiments, the change in the polymer's microenvironment is preferably induced at the time of, or subsequent to the time the device is inserted or implanted in the subject, so as to trigger the release of the API from the device at the desired site of action.

### 7. EXAMPLES (non-inventive)

A solution containing 8.8 wt.% of paclitaxel (Ptx) and 91.2 wt.% styrene-isobutylene-styrene (SIBS) in a mixed solvent of toluene/tetrahydrofuran (95/5) is spray dried into particles with an average particle diameter of 12 microns. The particles are collected and combined with gaseous N2 to form a fluidized bed. The fluidized bed is circulated at a temperature less than 5 °C to prevent particle agglomeration. A stent is immersed in the fluidized bed and heated to a maximum temperature of 50 °C by illuminating it with a near Infra-red laser beam that is absorbed by the stent material and not the SIBS/Ptx particles. When a cold SIBS/Ptx particle impinges on the warm stent surface the polymer heats up, becomes tacky, and adheres to the stent surface. The stent is removed from the fluidized bed after a specific time that is known by previous experiments to allow the required number of particles per area to deposit on the stent surface.

In an alternative embodiment, poly(lactide-co-glycolide) (PLGA)is used in place of SIBS to form particles which are bioabsorbable.

## Claims

1. A medical device comprising a surface and a plurality of microparticles comprising an active pharmaceutical ingredient (API) and a thermosetting polymer, wherein the microparticles are disposed on said surface in the absence of a continuous phase coating by heating the surface and contacting the microparticles with the surface.

2. The medical device of claim 1, wherein said plurality of microparticles is disposed on said surface at a concentration of from 0.2 to 50 µg/mm2 on the basis of the API.

3. The medical device of claim 1, wherein the API comprises at least 5 wt.-% of said microparticles.

4. The medical device of claim 1, wherein the medical device is a stent.

5. A method for making a coated medical device, said method comprising the following
(a) placing microparticles comprising an active pharmaceutical ingredient (API) and a thermosetting polymer in a fluidized bed chamber;
(b) pre-treating a surface of the medical device to promote adhesion between the surface and the microparticles by heating the surface; and
(c) contacting the pre-treated surface with the fluidized bed to dispose the microparticles on the surface, wherein the microparticles are disposed on said surface in the absence of a continuous phase coating.

6. The method of claim 5, wherein the microparticles are circulated with an inert gas during said contacting step (c).

7. The method of claim 5, wherein the API comprises at least 5 wt.-% of said microparticles.

8. The method of claim 5, wherein the method further comprises curing the coated medical device after said contacting step (c).

9. The method of claim 5, wherein the pre-treating step (b) comprises masking a portion of said surface with a masking agent.

10. The method of claim 5, wherein the medical device is a stent.
- 2-

## Patentansprüche

1. Medizinische Vorrichtung, umfassend eine Oberfläche und eine Vielzahl von Mikropartikeln, die einen pharmazeutischen Wirkstoff (API) und ein wärmehärtendes Polymer umfassen, wobei die Mikropartikel ohne Vorhandensein einer Beschichtung mit kontinuierlicher Phase durch Erhitzen der Oberfläche und Inkontaktbringen der Mikropartikel mit der Oberfläche auf der Oberfläche angeordnet werden.

2. Medizinische Vorrichtung gemäß Anspruch 1, wobei die Vielzahl von Mikropartikeln mit einer Konzentration von 0,2 bis 50 µg/mm² auf der Grundlage des API auf der Oberfläche angeordnet sind.

3. Medizinische Vorrichtung gemäß Anspruch 1, wobei der API wenigstens 5 Gew.-% der Mikropartikel umfasst.

4. Medizinische Vorrichtung gemäß Anspruch 1, wobei die medizinische Vorrichtung ein Stent ist.

5. Verfahren zum Herstellen einer beschichteten medizinischen Vorrichtung, wobei das Verfahren Folgendes umfasst:
(a) Anordnen von Mikropartikeln, die einen pharmazeutischen Wirkstoff (API) und ein wärmehärtendes Polymer umfassen, in einer Wirbelschichtkammer;
(b) Vorbehandeln einer Oberfläche der medizinischen Vorrichtung, um Haftung zwischen der Oberfläche und den Mikropartikeln zu fördern, durch Erhitzen der Oberfläche; und
(c) Inkontaktbringen der vorbehandelten Oberfläche mit der Wirbelschicht, um die Mikropartikel auf der Oberfläche anzuordnen, wobei die Mikropartikel ohne Vorhandensein einer Beschichtung mit kontinuierlicher Phase auf der Oberfläche angeordnet werden.

6. Verfahren gemäß Anspruch 5, wobei die Mikropartikel während des Schritts des Inkontaktbringens (c) mit einem inerten Gas zirkuliert werden.

7. Verfahren gemäß Anspruch 5, wobei der API wenigstens 5 Gew.-% der Mikropartikel umfasst.

8. Verfahren gemäß Anspruch 5, wobei das Verfahren ferner Härten der beschichteten medizinischen Vorrichtung nach dem Schritt des Inkontaktbringens (c) umfasst.

9. Verfahren gemäß Anspruch 5, wobei der Vorbehandlungsschritt (b) Maskieren eines Teils der Oberfläche mit einem Maskierungsmittel umfasst.

10. Verfahren gemäß Anspruch 5, wobei die medizinische Vorrichtung ein Stent ist.

## Revendications

1. Dispositif médical comprenant une surface et une pluralité de microparticules comprenant un ingrédient pharmaceutique actif (API) et un polymère thermodurcissable, où les microparticules sont disposées sur ladite surface en l'absence d'un revêtement en phase continue par chauffage de la surface et mise en contact des microparticules avec la surface.

2. Dispositif médical selon la revendication 1, dans lequel ladite pluralité de microparticules est disposée sur ladite surface à une concentration de 0,2 à 50 µg/mm² en fonction de l'API.

3. Dispositif médical selon la revendication 1, dans lequel l'API comprend au moins 5 % en poids desdites microparticules.

4. Dispositif médical selon la revendication 1, dans lequel le dispositif médical est une endoprothèse vasculaire.

5. Procédé de fabrication d'un dispositif médical revêtu, ledit procédé comprenant les étapes suivantes:
(a) placer des microparticules comprenant un ingrédient pharmaceutique actif (API) et un polymère thermodurcissable dans une chambre à lit fluidisé ;
(b) prétraiter une surface du dispositif médical pour favoriser l'adhérence entre la surface et les microparticules en chauffant la surface ; et
(c) mettre en contact la surface prétraitée avec le lit fluidisé pour disposer les microparticules sur la surface, où les microparticules sont disposées sur ladite surface en l'absence de revêtement en phase continue.

6. Procédé selon la revendication 5, dans lequel les microparticules sont mises en mouvement par un gaz inerte au cours de ladite étape de mise en contact (c).

7. Procédé selon la revendication 5, dans lequel l'API comprend au moins 5 % en poids desdites microparticules.

8. Procédé selon la revendication 5, dans lequel le procédé comprend en outre de traiter par cuisson le dispositif médical revêtu après ladite étape de mise en contact (c).

9. Procédé selon la revendication 5, dans lequel l'étape de prétraitement (b) comprend de masquer une partie de ladite surface avec un agent de masquage.

10. Procédé selon la revendication 5, dans lequel le dispositif médical est une endoprothèse vasculaire.
